# EUROPÄISCHE PATENTSCHRIFT

(11) **EP 3 255 029 B1**
(45) Veröffentlichungstag und Bekanntmachung des Hinweises auf die Patenterteilung: **14.11.2018**
(21) Anmeldenummer: 16173939.6
(22) Anmeldetag: 10.06.2016
(51) Int. Cl.: C07C 2/08, C07C 11/02, C07C 11/08, C07C 11/107

(54) **OLIGOMERISIERUNG VON ETHEN IN ÜBERKRITISCHER FAHRWEISE**
ETHYLENE OLIGOMERISATION UNDER SUPERCRITICAL CONDITIONS
OLIGOMÉRISATION DE L'ÉTHYLÈNE DANS DES CONDITIONS SUPERCRITIQUES

(43) Veröffentlichungstag der Anmeldung: 13.12.2017
(73) Patentinhaber: Evonik Degussa GmbH, 45128 Essen (DE)
(72) Erfinder: REEKER, Helene, 44227 Dortmund (DE); STOCHNIOL, Guido, 45721 Haltern am See (DE); MASCHMEYER, Dietrich, 45657 Recklinghausen (DE); PEITZ, Stephan, 45739 Oer-Erkenschwick (DE); SCHALLENBERG, Jörg, 46286 Dorsten (DE)

(56) Entgegenhaltungen:
- EP-A1- 1 085 001
- WO-A1-95/14647
- WO-A2-2010/117539

## Beschreibung

Die Erfindung befasst sich mit der Oligomerisierung von überkritischem Ethen.

Kohlenwasserstoffe sind chemische Verbindungen, die ausschließlich aus Kohlenstoff und Wasserstoff bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet.

In der organischen Chemie werden Kohlenwasserstoffe häufig nach der Anzahl ihrer Kohlenstoffatome pro Molekül bezeichnet, indem der jeweiligen Substanzklasse das Präfix Cₙ vorangestellt wird. Dabei bezeichnet n die jeweilige Anzahl der Kohlenstoffatome in einem Molekül. So sind C₄-Olefine zu verstehen als Substanzen der Klasse der Alkene mit vier Kohlenstoffatomen. C₈-Olefine weisen dementsprechend acht Kohlenstoffatome pro Molekül auf. Soweit im Folgenden das Präfix Cₙ₊ verwendet wird, ist von einer Substanzklasse die Rede, die mehr als n Kohlenstoffatome pro Molekül aufweist. Ein C₄₊-Olefin hat demnach mindestens fünf Kohlenstoffatome.

Das einfachste Olefin ist Ethen (Ethylen). Es weist zwei Kohlenstoffatome auf. Ethen stellt eine bedeutsame Grundchemikalie dar und wird daher in großen Mengen hergestellt. Dies geschieht meist durch Spaltung von Naphtha. Darüber hinaus kann es durch Dehydrierung von Ethan gewonnen werden, welches wiederum ein Bestandteil des Erdgases ist. Aufgrund zunehmender Erschließung von unkonventionellen Erdgasquellen und abnehmender Erdöl-Förderung nimmt der Anteil an auf Erdgas basierendem Ethen stetig zu. Die physikalischen Eigenschaften von Ethen und dessen Herstellung sind beschrieben in:
Zimmermann, Heinz und Walzl, Roland: Ethylene. Ullmann's Encyclopedia of Industrial Chemistry (2009).

Unter der Oligomerisierung versteht man die Reaktion von Kohlenwasserstoffen mit sich selbst, wobei entsprechend längerkettige Kohlenwasserstoffe, die so genannten Oligomere, entstehen. Olefine mit zwei bis acht Kohlenstoffatomen lassen sich recht gut oligomerisieren.

So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt. Verbinden sich hingegen drei Olefine mit drei Kohlenstoffatomen miteinander (Trimerisierung), entsteht ein Olefin mit neun Kohlenstoffatomen.

Werden n-Butene - das sind Olefine mit vier Kohlenstoffatomen - einer Oligomerisierung unterworfen, entstehen dabei im Wesentlichen Olefine mit acht Kohlenstoffatomen (genauer gesagt: Dibuten) und dazu Olefine mit zwölf Kohlenstoffatomen (C₁₂-Olefine, "Tributen"), sowie in einem geringeren Umfang Olefine mit mehr als zwölf Kohlenstoffatomen (C₁₂+-Olefine).

Abhängig von der Anzahl der Kohlenstoffatome und dem so genannten Verzweigungsgrad werden die Oligomere für die Herstellung von Weichmacher-Estern, Detergentien oder als Kraftstoffzusatz verwendet:
Friedlander, Ward, Obenaus, Nierlich, Neumeister: Make plasticizer olefins via n-butene dimerization. Hydrocarbon Processing, February 1986, Seiten 31 bis 33.
F. Nierlich: Oligomerize for better gasoline. Hydrocarbon Processing, February 1992, Seiten 45 bis 46.

Die Oligomerisierung von Ethen wird erst durch Einsatz von Katalysatoren industriell praktikabel. Industrielle Prozesse zur Oligomerisierung von Ethen können grob unterschieden werden in homogen katalysierte Verfahren und heterogen katalysierte Verfahren.

In homogen katalysierten Verfahren wird der Katalysator in dem Reaktionsgemisch gelöst. Die Reaktion erfolgt in der flüssigen Phase, in der sich auch der gelöste Katalysator befindet. Vorteil der homogenen Katalyse ist die hohe Effizienz der Reaktion; Nachteil ist, dass der gelöste Katalysator aufwendig aus dem Reaktionsgemisch abgetrennt werden muss. Sofern der Katalysator sehr preiswert ist (wie z.B. Triethylaluminium) und Katalysatorreste das Oligomerisat nicht unzulässig verschmutzen, kann auf eine Abtrennung verzichtet werden. Beispiele für homogen katalysierte Oligomerisierung von Ethen zeigen WO2005/123633 und US2013/0066128.

In heterogen katalysierten Verfahren liegt der Katalysator als ein Festkörper im Reaktor vor. Das durch den Reaktor strömende Ethen kommt mit dem festen Katalysator in Kontakt. Das Oligomerisat wird aus dem Reaktor abgezogen und der feste Katalysator bleibt im Reaktor zurück. Vorteil der heterogen katalysierten Prozesse ist, dass die aufwendige Katalysatorabtrennung entfällt und das Reaktionsprodukt nicht durch Abbauprodukte des Katalysators verunreinigt wird. Nachteil ist, dass der Katalysator mit der Zeit desaktiviert, insbesondere dadurch, dass sich Polyethylen auf dem Katalysator niederschlägt und die katalytisch aktiven Kontakte blockiert. Die Standzeit des Katalysators ist daher begrenzt. Ein desaktivierter Katalysator muss entweder ausgetauscht oder regeneriert werden.

Die heterogene Oligomerisierung kann wahlweise in der flüssigen oder in der gasförmigen Phase erfolgen. Die Flüssigphasen-Oligomerisierung hat den Vorteil, dass das Reaktionsgemisch eine größere Dichte aufweist und dadurch das Apparatevolumen effizienter ausgenutzt wird. Vorteil der Gasphasen-Oligomerisierung von Ethen sind die geringeren Drücke, welche die Apparate preiswerter und einfacher zu beherrschen machen.
Daneben gibt es erste Ansätze Ethen im überkritischen Zustand zu oligomerisieren:
WO1995/14647A1 beansprucht ein Verfahren zum Oligomerisieren von unverzweigten C₂ bis C₆ Olefinen zu Dimeren, Trimeren und Tetrameren mittels eines Festbettkatalysators, bei dem man die Oligomerisierung in einer Reaktionszone bei überkritischer Temperatur und überkritischem Druck der eingesetzten Olefine durchführt und keine zusätzlichen Lösungsmittel verwendet, die sich in der Reaktionszone nicht im überkritischen Zustand befinden. Der eingesetzte Katalysator enthält Titandioxid, Aluminiumoxid, Nickeloxid und Siliciumdioxid sowie herstellungsbedingt Alkalimetalloxid. Dieser Katalysator eigne sich hervorragend zur Oligomerisierung von Butenen in Buten/Butan-Gemischen. Seine Eignung für die Oligomerisierung von Ethen erörtert WO1995/14647A1 indes nicht. Konkrete Angaben zu den einzusetzenden Lösemitteln (Stoff, Konzentration, thermodynamischer Zustand) fehlen. Der inhaltliche Schwerpunkt dieser Druckschrift liegt vielmehr auf der Herstellung des Festbettkatalysators als auf der Reaktionsführung.
Aus der WO2010/117539A2 ist ein Verfahren zur Oligomerisierung von Ethen bekannt, welches in einem stark verunreinigten Einsatzgemisch daher kommt. Das Einsatzgemisch stammt aus einem Fluid-katalytischen Naphtha-Cracker (FCC) und enthält als Hauptbestandteile Methan und/oder Ethan, größere Mengen Wasserstoff und Stickstoff sowie Kohlenmonoxid, Kohlendioxid und Schwefelwasserstoff als Verunreinigungen. Beiläufig wird erwähnt, dass der Druck oberhalb des kritischen Drucks von reinem Ethen durchgeführt werden könne; hinsichtlich der kritischen Temperatur von Ethen werden keinerlei Angaben gemacht. Die Reaktion erfolge im Wesentlichen in der Gasphase. Es ist erklärtes Ziel der WO2010/117539A2 verunreinigte Ethen-haltige Abgase in flüssige Kraftstoffe umzuwandeln, welche dem Diesel- bzw. Benzin-Pool zugeschlagen werden können. Da der Heizwert der Kraftstoffe mit der Anzahl der C-Atome der darin enthaltenen Kohlenwasserstoffe steigt, macht es Sinn ein solches Verfahren in Richtung der Produktion von längerkettigen Oligomeren zu optimieren. Tatsächlich enthält das in WO2010/117539A2 beschriebene Oligomerisat eine große Menge an Olefinen mit zehn oder mehr Kohlenstoffatomen.
Optimiert auf die Herstellung von Olefinen mit vier bis acht Kohlenstoffatomen aus Ethen sind demgegenüber die Verfahren, die in einigen zum Zeitpunkt der Anmeldung noch unveröffentlichten Anmeldungen niedergelegt sind. In besagten Anmeldungen wird Ethen in Gegenwart mindestens eines inerten Lösemittels oligomerisiert. So wird in US 15/000,807 (US2016/0207248 A1) bzw. in EP 16151490.6 (EP3045439 A1) n-Hexan als Lösemittel eingesetzt, währenddessen in US 15/000,837 (US2016/0207849 A1) bzw. in EP 15151624.2 (EP3045438 A1) Propan bzw.

Isobutan als Lösemittel bevorzugt wird. In allen Fällen liegt sowohl das Ethen als auch das Lösemittel im flüssigen Zustand vor.

Ebenfalls unveröffentlicht war damals noch die europäische Anmeldung 16158044.4 (EP3064274 A1) bzw. US 15/056, 147 (US2016/0257630 A1), welche die in-situ Regeneration eines in der Ethen-Oligomerisierung eingesetzten festen Katalysators beschreibt. Die Regeneration erfolgt mit Hilfe eines flüssigen Spülmediums, welches im Oligomerisierungs-Betrieb als Lösemittel für das Ethen dient. Als Spülmedium bzw. Lösemittel werden Propan, Isobutan, Pentan, Cyclopentan, Hexan, Cyclohexan, Heptan oder Cycloheptan vorgeschlagen. Sowohl im Oligomerisierungs-Betrieb als auch im Regenerations-Betrieb befindet sich das Lösemittel bzw. das Spülmedium im flüssigen Zustand.
In Hinblick auf diesen Stand der Technik besteht die der Erfindung zu Grunde liegenden Aufgabe darin, ein Verfahren zur Oligomerisierung von Ethen anzugeben, welches ermöglicht die Raum-Zeit-Ausbeute deutlich zu steigern. Daneben soll eine hohe Selektivität in Richtung der Dimere, Trimere und Tetramere des Ethens erreicht werden. Olefine mit zehn oder mehr Kohlenstoffatome (C₁₀₊-Olefine) sollen hingegen kaum gebildet werden. Darüber hinaus soll das Verfahren effizient sein, insbesondere soll das Apparatevolumen bestmöglich ausgenutzt werden. Ein weiteres wichtiges Ziel ist die Verlängerung der Standzeit des Katalysators.
Gelöst werden diese Aufgaben durch ein heterogen katalysiertes Verfahren, bei dem die Oligomerisierung des Ethens in Gegenwart eines Inertmediums durchgeführt wird, wobei sowohl das Inertmedium als auch das Ethen im überkritischen Zustand vorliegen. Bei dem Inertmedium handelt es sich um ein Alkan oder um ein Cycloalkan, welches mindestens drei und maximal sieben Kohlenstoffatome aufweist. Entscheidend ist, dass Druck und Temperatur des aus Ethen und Inertmedium gebildeten Gemisches in Hinblick auf den Anteil von Ethen in dem Gemisch so gewählt sind, dass Ethen und Inertmedium im überkritischen Zustand vorliegen. Schließlich ist der Gewichtsanteil des Inertmediums an dem Gemisch größer zu wählen als der Gewichtsanteil des Ethens an dem Gemisch.
Ein entsprechendes Verfahren ist Gegenstand der Erfindung und niedergelegt in Anspruch 1.
Ein Wesentlicher Aspekt der Erfindung ist, dass Ethen mit dem Inertmedium zu vermischen und die Bedingungen bei der Reaktion so einzustellen, dass sowohl Ethen und Inertmedium überkritisch sind. Im überkritischen Zustand ist nämlich die Löslichkeit für Ethen in dem Inertmedium größer, sodass mehr Ethen in dem überkritischen Inertmedium gelöst werden als in einem flüssigen Lösemittel. Die Prozessführung im überkritischen Zustand ermöglicht es daher, einen deutlich höheren Anteil Ethen in einer homogenen Mischung aus Ethen und Inertmedium einzusetzen, als es aufgrund der thermodynamischen Löslichkeitsbeschränkung in einem rein flüssigen Kohlenwasserstoffstrom möglich ist. Auf diese Weise wird die Raum-Zeit-Ausbeute deutlich gesteigert. Da eine größere Menge Ethen in den Reaktor geleitet werden kann, lässt sich im Ergebnis das Apparatevolumen verglichen mit einem Flüssigphasen-Verfahren besser auszunutzen.

In diesem Zusammenhang soll darauf hingewiesen werden, dass im überkritischen Zustand strenggenommen nicht von einer Lösung gesprochen werden kann; vielmehr koexistieren zwei überkritische Phasen innerhalb des Gemisches, nämlich die aus Ethen und die aus Inertmedium. Da der Gewichtsanteil des Inertmediums in dem Gemisch aber größer ist als die des Ethens, wirkt das Inertmedium analog wie ein Lösemittel in einem Flüssigphasen-Prozess. Insoweit ist auch der soeben angestellte Vergleich der "Löslichkeit" von überkritischem Ethen in überkritischem Inertmedium mit der Löslichkeit von flüssigen Ethen in einem flüssigen Lösemittel zulässig.

Die Löslichkeit im überkritischen Inertmedium ist nicht nur für Ethen erhöht, sondern auch für unerwünschte Nebenprodukte der Reaktion wie etwa Polyethylen. Polyethylen ist deshalb unerwünscht, weil es auf dem Katalysator und den Apparaten einen wachsartigen Niederschlag bildet, welcher die Aktivität des Katalysators senkt und die Apparate verstopft. Da sich Polyethylen hervorragend in überkritischen Alkanen oder Cycloalkanen mit drei bis sieben Kohlenstoffatome löst, wird das Polyethylen effektiv von dem Inertmedium fortgespült und der Katalysator und die Apparate sauber gehalten. Die daraus resultierende verlängerte Standzeit des Katalysators belegen die Versuche.

Die Tatsache, dass in dem hier beschriebenen Verfahren ein Alkan oder ein Cycloalkan mit drei bis sieben Kohlenstoffatome als Inertmedium eingesetzt wird hat den physikalischen Grund , dass diese Stoffe im Gemisch mit Ethen mit vertretbarem Aufwand in den überkritischen Zustand versetzt werden können; die erforderlichen Temperaturen und Drücke werden nicht zu groß: Die kritischen Drücke der genannten reinen Komponenten bewegen sich zwischen 27.4*10⁵ Pa für n-Heptan und 50.4*10⁵ Pa für Ethen. Die kritischen Temperaturen liegen zwischen 9.2°C für Ethen und 331°C für Cycloheptan. Es bieten sich also vor allem Inertmedien mit niedriger Molmasse an, da diese eine niedrigere kritische Temperatur aufweisen und demnach die kritischen Prozessparameter mit weniger Energieaufwand zu erreichen sind. Deswegen ist es einfacher, das Gemisch aus Ethen und Inertmedium über den gesamten Reaktionszeitraum hinweg im überkritischen Zustand zu halten. Es ist nämlich zu verhindern, dass eine der beiden Komponenten noch im Reaktor den kritischen Zustand verlässt und eine flüssige oder gasförmige Phase bildet.

Ebenfalls einfach ist das Bereitstellen eines Gemisches aus Ethen mit einem C₃- bis C₇- Alkan bzw. Cycloalkan im überkritischen Zustand, bevor dieses mit dem heterogenen Katalysator kontaktiert wird. Hier gibt es nämlich zwei Möglichkeiten:
a) Inertmedium wird durch Erhöhung von Druck und/oder Temperatur in den überkritischen Zustand überführt und Ethen wird in das überkritische Inertmedium unter Erhalt des überkritischen Gemisches eindosiert;
b) Inertmedium und Ethen werden zu dem Gemisch vermischt, und das Gemisch durch Erhöhung von Druck und/oder Temperatur in den überkritischen Zustand überführt.

Beide Alternativen sind verfahrenstechnisch leicht umzusetzen.

Aus chemischer Sicht sind die Alkane und Cycloalkane als Inertmedium prädestiniert, da sie - verglichen mit dem Ethen - praktisch keine Reaktivität aufweisen und somit an der Oligomerisierung nicht teilnehmen. Sie verhalten sich in der Reaktion inert. Darüber hinaus desaktivieren sie den Katalysator nicht nennenswert.

Sofern in der Oligomerisierung weitere Stoffe außer Ethen und Inertmedium anwesend sind, etwa Produktionsrückstände aus deren Herstellprozessen, dürfen diese Begleitstoffe durchaus eine flüssige oder gasförmige Phase im Reaktionsgemisch bilden, da sie ja nicht an der Reaktion teilnehmen. Die Produkte der Reaktion, also die Oligomere des Ethens, dürfen ebenfalls eine flüssige oder gasförmige Phase bilden. Sie werden dennoch von dem überkritischen Inertmedium aus dem Reaktor ausgetragen. Wenn solche Begleitstoffe bei den gewählten Reaktionsbedingungen ebenfalls überkritisch sind, ist das gleichwohl unschädlich.

Im Ergebnis muss lediglich das Gemisch bestehend aus Ethen und Inertmedium überkritisch sein. Da die Grenze des überkritischen Zustands des Gemisches von Druck, Temperatur und Mengenverhältnis von Ethen zu Inertmedium vorgegeben sind, müssen Druck und Temperatur in Hinblick auf den Anteil von Ethen in dem Gemisch so eingestellt werden, dass Ethen und Inertmedium im überkritischen Zustand vorliegen.

Sofern das Reaktionsgemisch mehrere Alkane und/oder Cycloalkane mit drei bis sieben Kohlenstoffatomen enthält, genügt es wenn zumindest eine dieser Substanzen bei den gewählten Bedingungen überkritisch ist und mithin als Inertmedium dient.

Die thermodynamischen Eigenschaften der Gemische aus Ethen mit den hier angegebenen Inertmedien lassen sich auf Grundlage von verfügbaren Stoffdaten der Reinsubstanzen mit Hilfe handelsüblicher Simulationssoftware bestimmen. Für die Festlegung der Reaktionsbedingungen wurde hier die Simulationssoftware Aspen Properties V7.3, Firma Aspen Technology, Inc. verwendet, die thermodynamischen Eigenschaften der Reinsubstanzen stammen aus der Stoffdatenbank APV73 mit PURE25, welches auf dem Release der DIPPR Datenbank (Januar 2010) basiert.

Die folgenden Substanzen kommen als Inertmedium in die engere Auswahl: Propan, Isobutan, n-Butan, n-Pentan, Isopentan, n-Hexan, n-Heptan, Cyclopentan, Cyclohexan, 2-Methylpentan, 3-Methylpentan, 2-Methylhexan, 3-Methylhexan, Methylcyclopentan, Methylcyclohexan. Es genügt, eine dieser Substanzen als Inertmedium zu verwenden, es können auch mehrere dieser Substanzen vermischt werden. Bevorzugt wird aber nur genau eine dieser Substanzen als Inertmedium eingesetzt, da sich dann die thermodynamischen Eigenschaften des binären Gemisches aus Ethen und Inertmedium einfacher bestimmen lassen.

Wird als Inertmedium einzig Isobutan verwendet, sollte der Anteil des Ethens im Gemisch zu Beginn des Kontakts mit dem Katalysator zwischen 4 Gew.-% und 30 Gew.-% betragen, der Druck des Gemisches sollte zwischen 25*10⁵ Pa und 100*10⁵ Pa liegen, und die Temperatur des Gemisches sollte auf einen Wert zwischen 90 °C und 200 °C eingestellt sein. Diese drei Parameter dürfen freilich nicht unabhängig aus den angegeben Bereich ausgewählt werden sondern stets unter der Maßgabe, dass sowohl Ethen als auch Isobutan im überkritischen Zustand vorliegen. Werte-Tripel, welche diese Bedingung erfüllen findet die Simulationssoftware.

Je weniger Begleitstoffe neben dem Ethen und dem Inertmedium in der Oligomerisierung anwesend sind, desto einfacher ist es, den überkritischen Zustand des Ethens und des Inertmediums zu gewährleisten. Das in dem Prozess eingesetzte Ethen sollte möglichst frei von Verunreinigungen oder Begleitstoffen sein. Bevorzugt wird kommerziell verfügbares Ethen in der Reinheitsstufe "polymer grade" eingesetzt, dies hat typischerweise eine Reinheit von über 99.9 Gew.-%.

Sofern für das Verfahren das Gemisch aus Ethen und Inertmedium zusammen mit mindestens einem Begleitstoff als Eduktgemisch bereitgestellt wird, sollte das Eduktgemisch zum Beginn des Kontakts mit dem Katalysator die folgende, sich zu 100 Gew.-% ergänzende Zusammensetzung aufweist:

| | |
|---|---|
| Isobutan: | 70 Gew.-% bis 96 Gew.-%; |
| Ethen: | 4 Gew.-% bis 30 Gew.-%; |
| Summe aller Begleitstoffe: | mehr als 0 Gew.-% bis maximal 5 Gew.-% |

Die Summe aller Begleitstoffe umfasst alle Substanzen die zu Beginn des Kontakts des Eduktgemisches mit dem Katalysator anwesend sind außer das Isobutan und das Ethen.

Fachleuten fällt auf, dass das hier spezifizierte Eduktgemisch verglichen mit dem in WO2010/117539A2 eingesetzten Feed sehr sauber ist. Dies wirkt sich positiv auf die Produktzusammensetzung und die Prozessintensität aus.

Durch die mit dem Kontakt beginnende Oligomerisierung ist die Zusammensetzung des von Katalysator abgezogenen Produktgemisches deutlich komplexer als die des Eduktgemisches:

| | |
|---|---|
| Isobutan: | 70 Gew.-% bis 96 Gew.-%; |
| Ethen: | 0 Gew.-% bis 2 Gew.-%; |
| Olefine mit vier Kohlenstoffatomen: | 2.3 Gew.-% bis 21 Gew.-%; |
| Olefine mit sechs Kohlenstoffatomen: | 0.9 Gew.-% bis 7.2 Gew.-%; |
| Olefine mit acht Kohlenstoffatomen: | 0.1 Gew.-% bis 6.3 Gew.-%; |
| Olefine mit zehn Kohlenstoffatomen: | 0 Gew.-% bis 3 Gew.-%; |
| Olefine mit zwölf Kohlenstoffatomen: | 0 Gew.-% bis 2.7 Gew.-%; |
| Summe aller sonstigen Bestandteile: | 0 Gew.-% bis 5 Gew.-%. |

Selbstverständlich ergänzen sich sämtliche Bestandteile des Produktgemisches gemäß dieser Spezifikation zu 100 Gew.-%.

Diesmal fällt den fachkundigen Lesern auf, dass überwiegend Dimere, Trimere und Tetramere des Ethens gebildet werden, währenddessen die Selektivität der Reaktion in Richtung der höheren Oligomere mit zehn und mehr Kohlenstoffatomen gering ist. Das Produktgemisch unterscheidet sich demnach deutlich von dem in WO2010/117539A2 gebildeten Oligomerisat.

Ebenso wie Isobutan eignet sich n-Hexan hervorragend als Inertmedium. Aufgrund der thermodynamischen Eigenschaften des n-Hexans sollte der Anteil des Ethens im Gemisch zu Beginn des Kontakts mit dem Katalysator zwischen 5 Gew.-% und 30 Gew.-% betragen, der Druck des Gemisches auf einen Wert zwischen 25*10⁵ Pa und 100*10⁵ Pa eingestellt sein und die Temperatur des Gemisches sollte zwischen 90 °C und 250 °C liegen, wieder unter der Maßgabe, dass sowohl Ethen als auch n-Hexan sich im überkritischen Zustand befinden.

Das hier vorgestellte Verfahren zur Oligomerisierung von Ethen in überkritischer Fahrweise ist heterogen katalysiert. Als Katalysator kommt mithin ein Festkörper zum Einsatz. Ein geeigneter Katalysator enthält mindestens zwei Komponenten, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst. Ein Beispiel eines solchen Katalysators ist aus US2581228 bekannt. Auch WO1995/14647A1 beschreibt die Herstellung eines geeigneten Festbettkatalysators, wenngleich die Eignung dieses Katalysators für die Ethen-Oligomerisierung aus dieser Schrift nicht hervor geht.

Trotz des Einsatzes von überkritischem Inertmedium mit guter Löslichkeit für die den Katalysator desaktivierenden Substanzen ist die Standzeit des Katalysators begrenzt. Um einen schleichend desaktivierten Katalysator wieder voll einsatzfähig zu machen, ist eine Regeneration möglich. Die Regeneration erfolgt durch Spülen des Katalysators mit einem Spülmedium, welches allerdings flüssig sein sollte, um die Ablagerungen auf dem Katalysator zu entfernen. Die Regeneration kann *in-situ* erfolgen, also im Reaktor. Der Katalysator braucht dann nicht für die Regeneration ausgebaut werden, was die Betriebskosten deutlich senkt. Es wird dementsprechend zyklisch gewechselt zwischen einem Oligomerisierungs-Betrieb und einem Regenerations-Betrieb.

Gegenstand der Erfindung ist mithin auch eine Kombination des hier beschriebenen Verfahrens zur Oligomerisierung von Ethen in überkritischer Fahrweise mit einem Verfahren zur Regeneration des bei der Oligomerisierung eingesetzten Katalysators; mit den folgenden Merkmalen:
a) die Oligomerisierung erfolgt in einem Oligomerisierungs-Betrieb, in welchem Inertmedium und Ethen jeweils im überkritischen Zustand mit dem Katalysator kontaktiert werden;
b) die Regeneration erfolgt in einem Regenerations-Betrieb, in welchem der Katalysator in Abwesenheit von Ethen, Wasserstoff und Sauerstoff mit einem flüssigen Spülmedium gespült wird;
c) über die Zeit wird gewechselt zwischen dem Oligomerisierungs-Betrieb und dem Regenerations-Betrieb, sodass sich an einen zeitlich begrenzten Oligomerisierungs-Betrieb ein zeitlich begrenzter Regenerations-Betrieb anschließt, und an diesen wieder ein zeitlich begrenzter Oligomerisierungs-Betrieb;
d) der Katalysator befindet sich stets an demselben Ort, weswegen sowohl Oligomerisierungs-Betrieb als auch Regenerations-Betrieb an diesem Ort stattfinden;
e) der Ort, an dem sich der Katalysator befindet, wird mit positiver bzw. negativer Wärmenergie beaufschlagt, um ihm eine Vorgabe-Temperatur aufzuprägen, wobei die tatsächliche Temperatur am Katalysator zeitlich und örtlich begrenzt durchaus von der Vorgabe-Temperatur abweichen kann;
f) die Vorgabe-Temperatur im Regenerations-Betrieb ist niedriger als die Vorgabe-Temperatur im Oligomerisierungs-Betrieb;
g) der Druck an dem Ort, an dem sich der Katalysator befindet, ist im Regenerations-Betrieb niedriger als im Oligomerisierungs-Betrieb.

Eine besonders bevorzugte Weiterbildung dieser Kombination besteht darin, dass als Spülmedium und Inertmedium dieselbe Substanz verwendet wird, unter der Maßgabe, dass sich diese Substanz im Oligomerisierungs-Betrieb im überkritischen Zustand befindet und im Regenerations-Betrieb im flüssigen Zustand. Es wurde nämlich überraschend gefunden, dass sich die hier als geeignete Inertmedien beschriebenen Stoffe auch als Spülmedium eignen. Um zwischen Oligomerisierungs-Betrieb und Regenerations-Betrieb zu wechseln, braucht mithin nur der Ethen-Feed abgestellt und der Druck und die Temperatur gesenkt werden, um aus dem überkritischen Inertmedium ein flüssiges Spülmedium zu verwandeln. Der Betriebswechsel erfolgt dadurch sehr schnell und kann gut automatisiert werden.

Sofern als Spülmedium und Inertmedium Isobutan verwendet wird, und der Druck im Oligomerisierungs-Betrieb auf einen Wert zwischen 45*10⁵ Pa bis 55*10⁵ Pa eingestellt ist und die Temperatur im Oligomerisierungs-Betrieb auf einen Wert zwischen 125 °C und 155 °C eingestellt ist, werden im Regenerations-Betrieb Druck und Temperatur einfach abgesenkt, nämlich auf einen Druck zwischen 25*10⁵ Pa und 35*10⁵ Pa bzw. auf eine Temperatur zwischen 105 °C und 125 °C.

Auf diese Weise lässt sich mit wenig Aufwand der Katalysator rasch regenerieren. Da die Abstellzeiten in der industriellen Dauerproduktion einen immensen Kostentreiber darstellen, ist die hier beschriebene Kombination aus Oligomerisierung und Regeneration sehr wirtschaftlich.

Das Verfahren soll nun anhand von Beispielen näher erläutert werden. Die zugehörigen Graphen zeigen:
- Figur 1:: Ethylenoligomerisierung unter überkritischen Bedingungen (zu Beispiel 1)
- Figur 2:: Produktverteilung der Ethylenoligomerisierung unter überkritischen Bedingungen (zu Beispiel 1)
- Figur 3:: Ethylenoligomerisierung unter überkritischen Bedingungen und *in-situ-*Regeneration mittels flüssigen Isobutans im Wechsel (zu Beispiel 2)
- Figur 4:: Produktverteilung der Ethylenoligomerisierung unter überkritischen Bedingungen und *in*-*situ*-Regeneration mittels flüssigen Isobutans im Wechsel (zu Beispiel 2)
- Figur 5:: Beispiel einer Ethylenoligomerisierung unter überkritischen Bedingungen (zu Beispiel 3)
- Figur 6:: Produktverteilung der Ethylenoligomerisierung unter überkritischen Bedingungen (zu Beispiel 3)
- Figur 7:: Ethylenoligomerisierung unter überkritischen Bedingungen und *in-situ-*Regeneration mittels flüssigen Isobutans im Wechsel (zu Beispiel 4)
- Figur 8:: Produktverteilung der Ethylenoligomerisierung unter überkritischen Bedingungen und *in*-*situ*-Regeneration mittels flüssigen Isobutans im Wechsel (zu Beispiel 4)
- Figur 9:: Ethylenoligomerisierung unterhalb des kritischen Drucks der Zulaufmischung (zu Gegenbeispiel 5)
- Figur 10:: Produktverteilung der Ethylenoligomerisierung unterhalb des kritischen Drucks der Zulaufmischung (zu Gegenbeispiel 5)
- Figur 11:: Berechnete kritische Daten einer binären Mischung aus Isobutan und Ethen.

### Beispiel 1: Ethylenoligomerisierung in Isobutan unter überkritischen Bedingungen

12.6 g eines heterogenen Katalysators basierend auf Nickel, Titandioxid und Silica-Alumina (hergestellt nach WO9514647A1) wurden in einen außen mit Öl temperierten Rohrreaktor von 1 m Länge und 6 mm Innendurchmesser gefüllt. Anschließend wurde ein Gemisch aus 4 Massen-% bis 15 Massen-% Ethen und 85 Massen-% bis 96 Massen-% Isobutan nach einer Druckerhöhung auf 50 bar mit einem Gesamtmengenstrom von 120 g/h bei einer Temperatur von 140 °C kontinuierlich über den Katalysator gefahren (Raumgeschwindigkeit weight hourly space velocity WHSV = 9.5 h⁻¹ bzgl. des Gesamtzulaufs). Der Druck wurde dabei auf 50 bar konstant gehalten (50 bar entspricht 50*10⁵ Pa). Nach einer Zeit von etwa 23 h war ein Zustand erreicht, in dem sich der Umsatz nicht mehr änderte.

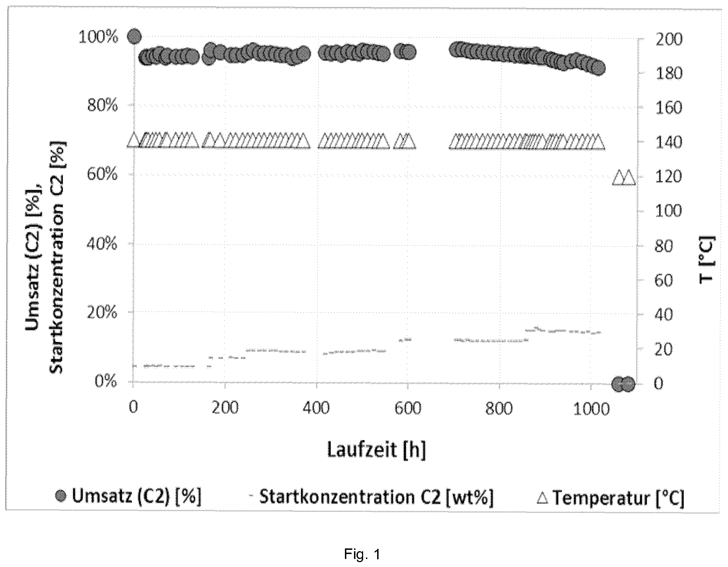

Figur 1 stellt den Umsatzverlauf einschließlich des Anteils des zugeführten Ethylens in Abhängigkeit von der Laufzeit der Reaktion dar. Der Ethen-Umsatz ist mit dem ausgefüllten Punkt ● markiert, die Startkonzentration des Ethens mit einem Strich - und die Temperatur mit einem Dreieck Δ

Über 600 h konnte ein relativ konstanter Ethylenumsatz von ca. 95% verzeichnet werden. Erst bei mehr als 12% Ethylen-Anteil im Feed erfolgte eine minimale Desaktivierung, die immer weiter fortschritt, so dass nach einer weiteren Laufzeit von knapp 300 h der Umsatz auf 91% abfiel und mit der Regeneration des Katalysators begonnen wurde.

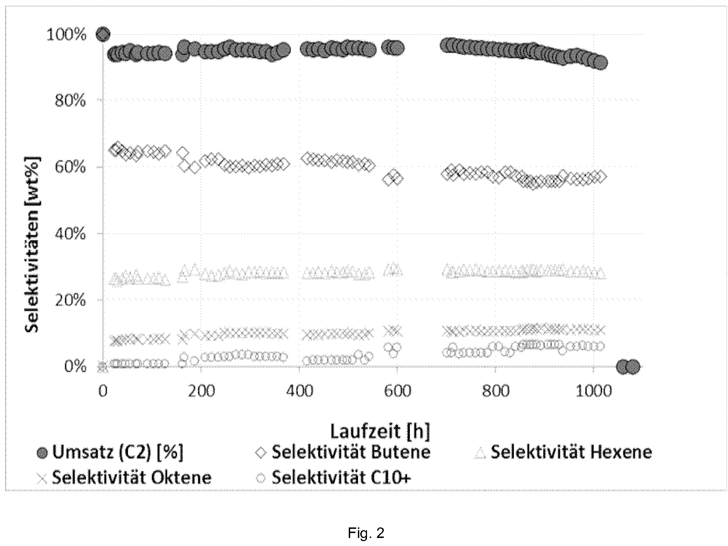

In Figur 2 wurden die erzielten Selektivitäten aufgetragen. Die Raute ◊ markiert die Selektivität hinsichtlich der Butene, das Dreieck Δ die Selektivität hinsichtlich der Hexene, das Kreuz X Selektivität hinsichtlich der Oktene und der Kreis ○ die Selektivität hinsichtlich der C₁₀₊-Olefine.

Wie aus Figur 2 ersichtlich ist, wurde über die gesamte Laufzeit des Versuches eine leicht sinkende Butenselektivität beobachtet, beginnend bei 65%. Sie erreichte bei etwa 1000 h Laufzeit einen Wert von etwa 57%. Die weiteren Produktfraktionen hingegen verzeichneten einen leichten Anstieg in ihrer Selektivität von jeweils etwa 2 bis 4 Prozentpunkten.

### Beispiel 2: Ethylenoligomerisierung unter überkritischen Bedingungen und in-situ Regeneration mittels des flüssigen Inertmediums Isobutan im Wechsel.

Zur Regeneration wurde der Versuch mit dem Katalysator aus Beispiel 1 fortgesetzt. Die Ethenzufuhr wurde unterbrochen und der Gesamtstrom zum Beschleunigen des Spülvorgangs auf 167 g/h erhöht. Die Temperatur wurde auf 120 °C und der Druck auf 30 bar (30*10⁵ Pa) herabgesetzt. Nach einem jeweiligen Regenerationsintervall von 75 h bis 98 h wurde der hinter der Anlage bei Raumtemperatur und 30 bar gehaltene feine Filter kontrolliert und bei Bedarf gegebenenfalls gewechselt. Dann wurde erneut Ethylen zugemischt, wobei der Gesamtstrom auf 120 g/h herabgesetzt wurde, während die Temperatur auf 140 °C und der Druck auf 50 bar erhöht wurden. Dieser Vorgang wurde mehrere Male wiederholt, sobald der Ethylenumsatz nach jeweils 130 h bis 188 h Laufzeit merklich gefallen war oder der verbaute Filter durch das Anzeigen einer Differenzdruckerhöhung auf Polymerablagerungen hinwies. Insgesamt wurden im Laufe des Versuchs 94 mg an Polyethylen hauptsächlich als feines Pulver aus dem Filter geborgen, was deutlich weniger als 0.01% der insgesamt eingesetzten Ethylenmenge ausmacht.

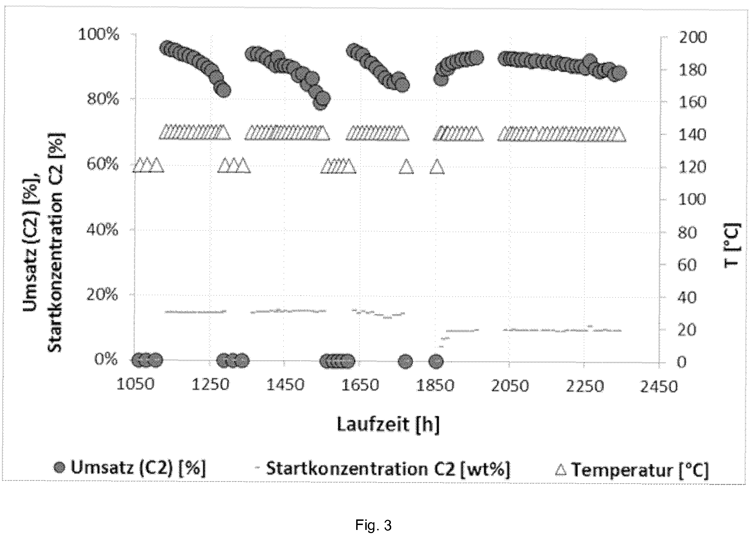

Figur 3 zeigt die beschriebenen Regenerationsintervalle in der flüssigen Phase (Intervalle bei 120 °C) im Wechsel mit Intervallen einer Ethenoligomerisierung unter überkritischen Bedingungen (Intervalle bei 140 °C). Der Ethen-Umsatz ist wieder mit dem ausgefüllten Punkt ● markiert, die Startkonzentration des Ethens mit einem Strich - und die Temperatur mit einem Dreieck Δ

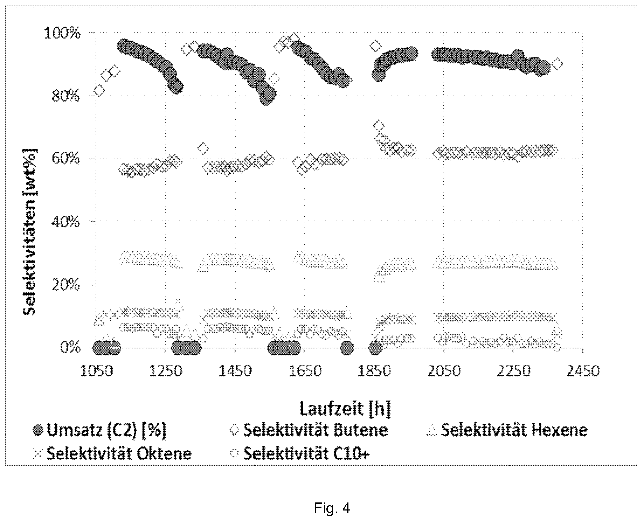

Die erzielten Selektivitäten wurden im Graph der Figur 4 aufgetragen. Die Raute ◊ markiert die Selektivität hinsichtlich der Butene, das Dreieck Δ die Selektivität hinsichtlich der Hexene, das Kreuz X Selektivität hinsichtlich der Oktene und der Kreis ○ die Selektivität hinsichtlich der C₁₀₊-Olefine. Figur 4 zeigt, dass durch die Regeneration in Verbindung mit der überkritischen Fahrweise eine Wiederherstellung der Anfangsselektivitäten aus Beispiel 1 ermöglicht wird. Die Butenselektivität erhöht sich im Verlaufe der Laufzeit wieder von 56 auf 63%, während die Selektivitäten der Hexen- und Oktenfraktion um je 2 Prozentpunkte absinkt. Besonders deutlich wird der Vorteil der überkritischen Fahrweise in der Fraktion C₁₀₊, der Selektivitätswert von deutlich von 6% auf 1% absinkt.

### Vergleich der Beispiele 1 und 2

Die Beispiele 1 und 2 zeigen, dass es möglich ist, eine Ethylenoligomerisierung unter überkritischen Bedingungen mit bis zu 15% Ethylenanteil langfristig bei sehr hohen Umsätzen von 80% bis 99% über eine beachtliche Laufzeit von über 2000 h hinweg kontinuierlich zu betreiben, wenn die Reaktion im Wechsel mit Regenerationsintervallen in der flüssigen Phase erfolgt, wobei die Regenerationsdauer und -häufigkeit von dem Anteil des Ethylens im Gemisch abhängt und im vorliegenden Beispiel ca. 14% der Gesamtlaufzeit beträgt. Die Regeneration kann dabei, wie gezeigt, ohne die Notwendigkeit eines Ausbaus des Reaktors, sondern einfach *in-situ* durch Spülen mit flüssigem Inertmedium nach dem Abstellen des Ethylens und Herabsenken von Druck und Temperatur realisiert werden. Des Weiteren wird aus Beispiel 2 ersichtlich, dass die überkritische Fahrweise in Kombination mit einer regelmäßigen Regeneration in der Lage ist, die sehr guten Anfangsselektivitäten wiederherzustellen und aufrecht zu erhalten.

### Beispiel 3: Ethylenoligomerisierung in Isobutan unter überkritischen Bedingungen.

19.3 g eines heterogenen Katalysators basierend auf Nickel und Silica-Alumina (hergestellt nach US 2581228) wurden in einen außen mit Öl temperierten Rohrreaktor von 1 m Länge und 6 mm Innendurchmesser gefüllt. Anschließend wurde ein Gemisch aus 4 Massen-% bis 17 Massen-% Ethen und 83 Massen-% bis 96 Massen-% Isobutan nach einer Druckerhöhung auf 50 bar mit einem Gesamtmengenstrom von 125 g/h bei einer Temperatur von 140 °C kontinuierlich mit einer Raumgeschwindigkeit (weight hourly space velocity WHSV) von 6.5 h⁻¹ bzgl. des Gesamtzulaufs über den Katalysator gefahren. Der Druck wurde dabei auf 50 bar konstant gehalten.

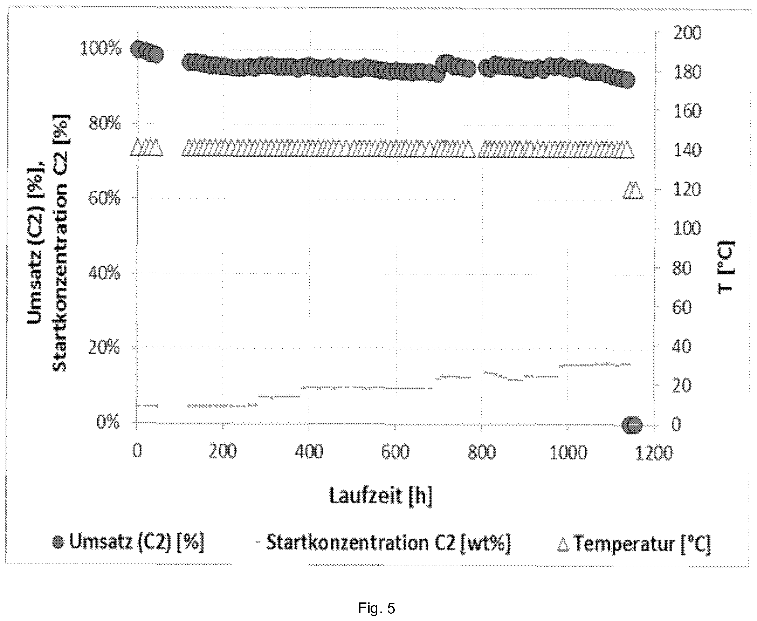

Figur 5 stellt den Umsatzverlauf in Abhängigkeit von der Laufzeit der Reaktion und des Anteils des zugeführten Ethylens im Reaktor dar. Der Ethen-Umsatz ist mit dem ausgefüllten Punkt ● markiert, die Startkonzentration des Ethens mit einem Strich - und die Temperatur mit einem Dreieck Δ

Nach einer anfänglichen leichten Desaktivierung des Katalysators konnte über 860 h ein relativ konstanter Ethylenumsatz von ca. 95% verzeichnet werden. Erst bei mehr als 13% Ethylen-Anteil im Zulauf erfolgte eine minimale Desaktivierung, die immer weiter fortschritt, so dass nach einer weiteren Laufzeit von knapp 144 h der Umsatz auf 92% abfiel und mit der Regeneration des Katalysators begonnen wurde.

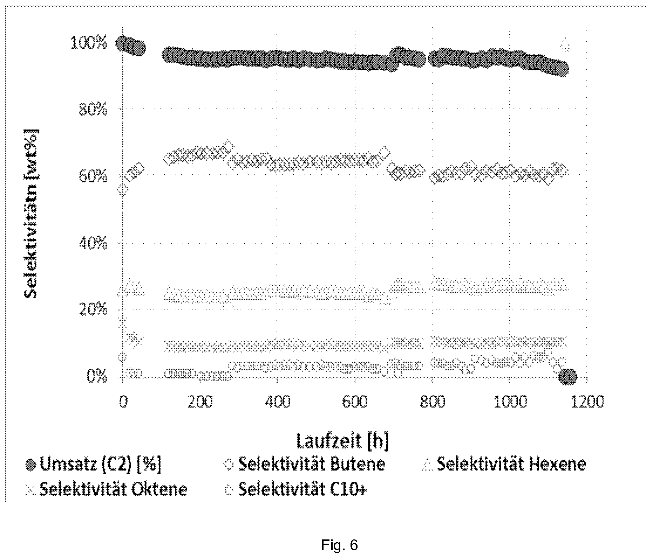

In Figur 6 sind die im Versuch 3 erzielten Selektivitäten aufgetragen. Die Raute ◊ markiert die Selektivität hinsichtlich der Butene, das Dreieck Δ die Selektivität hinsichtlich der Hexene, das Kreuz X Selektivität hinsichtlich der Oktene und der Kreis ○ die Selektivität hinsichtlich der C₁₀₊-Olefine. Aus Figur 6 ist ersichtlich, dass die Produktselektivitäten auf einem vergleichbaren Niveau wie in Beispiel 1 liegen. Auch ihre Entwicklung über die Laufzeit ist nahezu identisch.

### Beispiel 4: Ethylenoligomerisierung unter überkritischen Bedingungen und in-situ Regeneration mittels des flüssigen Inertmediums Isobutan im Wechsel

Zur Regeneration wurde der Versuch mit dem Katalysator aus Beispiel 3 fortgesetzt. Die Ethylenzufuhr wurde unterbrochen und der Gesamtstrom zum Beschleunigen des Spülvorgangs auf 167 g/h erhöht. Die Temperatur wurde auf 120 °C und der Druck auf 30 bar herabgesetzt. Nach einem jeweiligen Regenerationsintervall von 50 h bis 60 h wurde der hinter der Anlage bei Raumtemperatur und 30 bar gehaltene feine Filter kontrolliert und bei Bedarf gegebenenfalls gewechselt. Dann wurde erneut Ethylen zugemischt, wobei der Gesamtstrom auf 120 g/h herabgesetzt wurde, während die Temperatur auf 140 °C und der Druck auf 50 bar erhöht wurden. Das Gemisch variierte dabei zwischen 15 Massen-% und 25 Massen-% Ethen und 75 Massen-% bis 85 Massen-% Isobutan. Der Regenerationsvorgang wurde mehrere Male wiederholt, sobald der Ethylenumsatz nach jeweils 50 h bis 160 h Laufzeit merklich gefallen war oder der verbaute Filter durch das Anzeigen einer Differenzdruckerhöhung auf Polymerablagerungen hinwies. Insgesamt wurden im Laufe des Versuchs 350 mg an Polyethylen hauptsächlich als feines Pulver aus dem Filter geborgen, was deutlich weniger als 0.01% der insgesamt eingesetzten Ethylenmenge ausmacht.

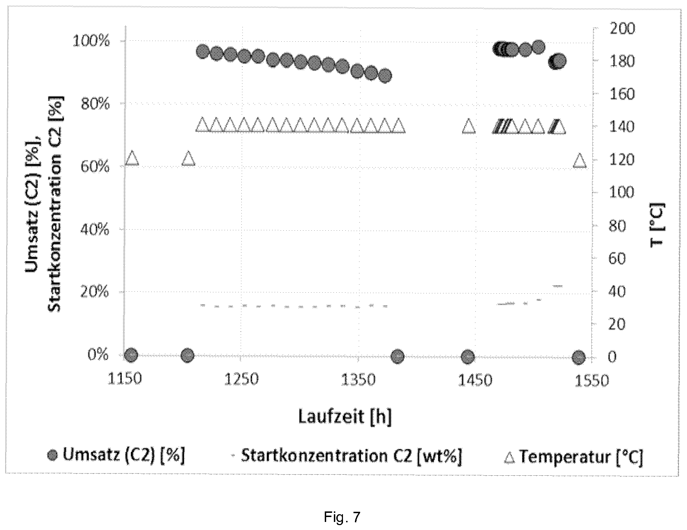

Figur 7 zeigt die beschriebenen Regenerationsintervalle in der flüssigen Phase (Intervalle bei 120 °C) im Wechsel mit Intervallen der Ethylenoligomerisierung unter überkritischen Bedingungen (Intervalle bei 140 °C). Der Ethen-Umsatz ist mit dem ausgefüllten Punkt ● markiert, die Startkonzentration des Ethens mit einem Strich - und die Temperatur mit einem Dreieck Δ

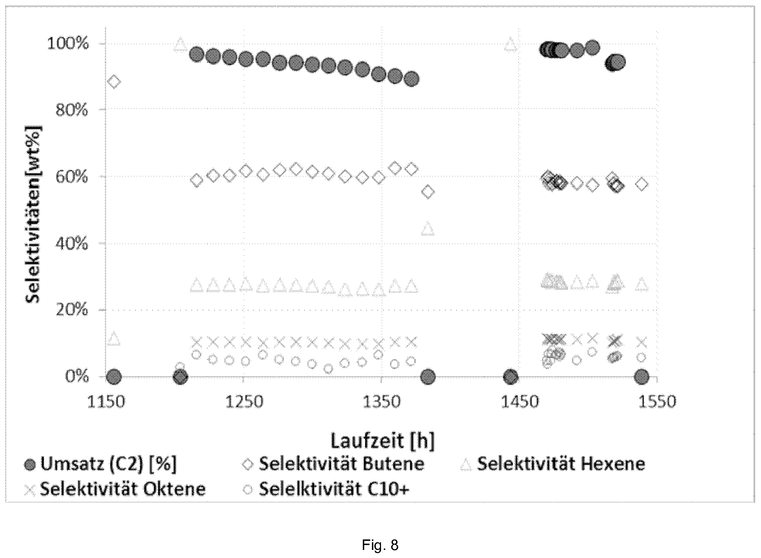

Figur 8 zeigt die zugehörige Produktverteilung zwischen den Regenrationszyklen. Die Raute ◊ markiert die Selektivität hinsichtlich der Butene, das Dreieck Δ die Selektivität hinsichtlich der Hexene, das Kreuz X Selektivität hinsichtlich der Oktene und der Kreis ○ die Selektivität hinsichtlich der C₁₀₊-Olefine. Wie zu erkennen ist, lässt sich mittels der Kombination von überkritischer Fahrweise und Regeneration auch bei sehr hohen Ethylenanteilen im Zulauf die Produktverteilung zumindest konstant halten.

### Diskussion der Beispiele 3 und 4

Die Beispiele 3 und 4 zeigen, dass es ebenso möglich ist, eine Ethylenoligomerisierung unter überkritischen Bedingungen mit mehr als 15% und mit bis zu 23% Ethylenanteil langfristig bei sehr hohen Umsätzen von 80 % bis 99% und konstanten Produktselektivitäten über eine beachtliche Laufzeit von über 1550 h hinweg kontinuierlich zu betreiben, wenn die Reaktion im Wechsel mit Regenerationsintervallen in der flüssigen Phase erfolgt, wobei die Regenerationsdauer und Häufigkeit der Regeneration von dem Anteil des Ethylens im Gemisch abhängt und im vorliegenden Beispiel ca. 4% der Gesamtlaufzeit beträgt. Die Regeneration kann dabei, wie gezeigt, ohne die Notwendigkeit eines Ausbaus des Reaktors, sondern einfach *in-situ* durch Spülen mit flüssigem Inertmedium nach dem Abstellen des Ethylens und Herabsenken von Druck und Temperatur realisiert werden.

### Gegenbeispiel 5: Ethylenoligomerisierung außerhalb des überkritischen Bereiches durch zu hohe Ethylenkonzentration

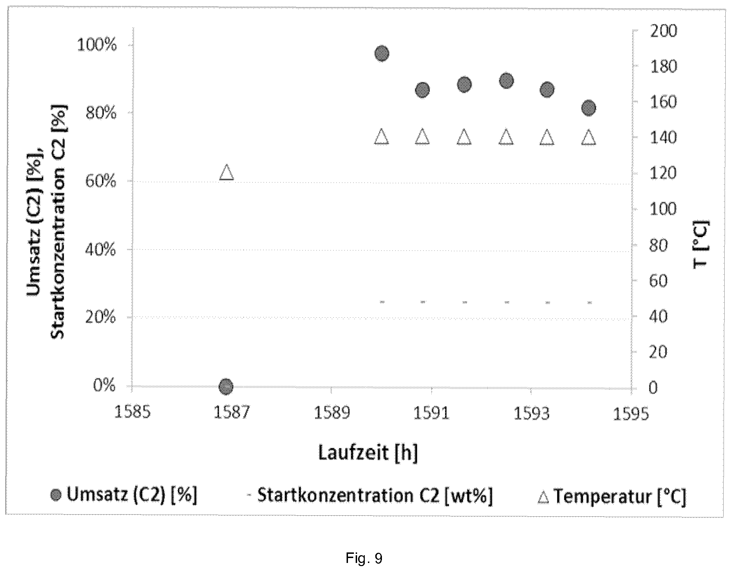

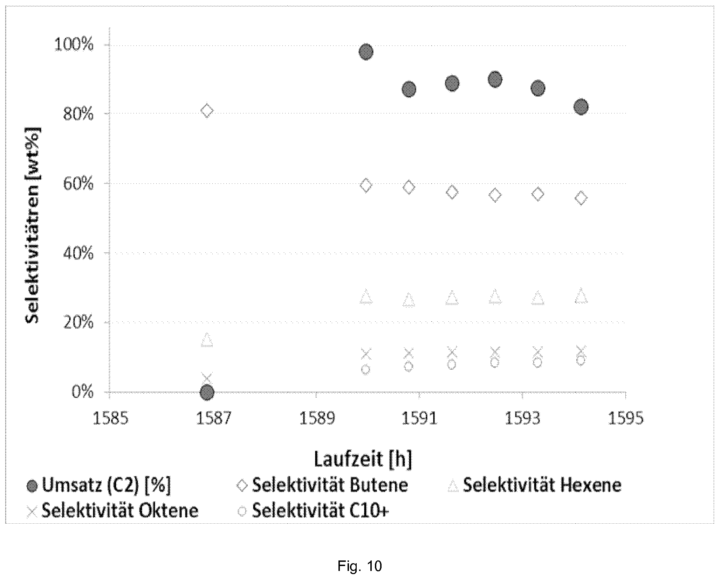

Nach einer Regenerationsphase von 50 h unter den gleichen Bedingungen wie in den vorherigen Beispielen wurde der Versuch mit dem Katalysator aus Beispielen 3 und 4 fortgesetzt. Dann wurde erneut Ethylen zugemischt, wobei der Gesamtstrom auf 120 g/h herabgesetzt wurde, während die Temperatur auf 140 °C und der Druck auf 50 bar erhöht wurden. Das Zulaufgemisch enthielt 25 Massen-% Ethen und 75 Massen-% Isobutan. Innerhalb von 5 h sank der Umsatz stark auf 82%. Diese schnelle Deaktivierung erfolgt durch Unterschreiten des kritischen Drucks, der bei diesem Anteil an Ethylen im Gemisch mit Isobutan bei etwa 54 bar liegt. Weiterhin sank in dieser sehr kurzen Zeitspanne parallel zum Umsatz auch die Selektivität zu Butenen von 60 % auf 56%, während die C1₀₊₋Fraktion deutlich von 6% auf 9% zunahm. Somit wurde gezeigt, dass die Einhaltung überkritischer Bedingungen essentiell für eine hohe Aktivität, Stabilität und Selektivität des Verfahrens ist. Die Figuren 9 und 10 zeigen das beschriebene Ethenoligomerisierungsintervall ab einer Laufzeit von 1590 h außerhalb der kritischen Phase. Die Markierungssymbole wurden entsprechend den anderen Graphen gewählt.

### Diskussion von Gegenbeispiel 5

Gegenbeispiel 5 zeigt, dass es entscheidend ist, eine Ethylenoligomerisierung unter überkritischen Bedingungen der Zulaufmischung zu betreiben. Gerade bei Unterschreiten des kritischen Druckes der Mischung erfolgt eine schnelle Deaktivierung des Katalysators unter gleichzeitigem starkem Selektivitätsverlust zu den kurzkettigen Wunschprodukten mit vier bis acht Kohlenstoffatomen innerhalb weniger Stunden.

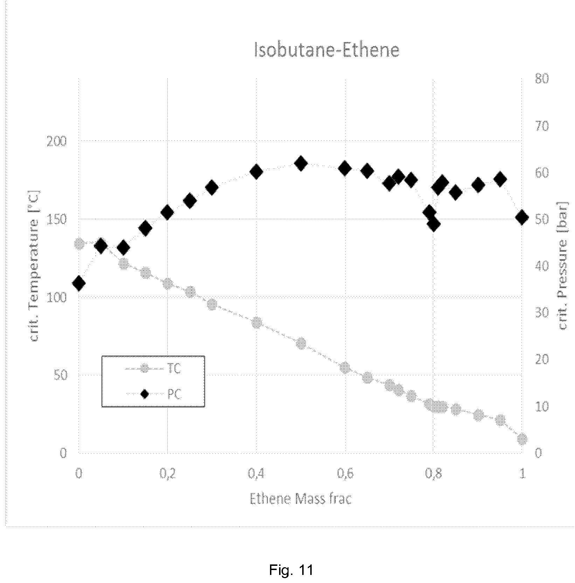

Mit zunehmendem Ethenanteil im Zulaufgemisch steigt der kritische Druck der Mischung mit einem Maximum um 50 Gewichts-% Ethen. Dieser Zusammenhang ist auch in Figur 11 ersichtlich. Die Berechnung der kritischen Drücke (Raute ◊) und kritischen Temperaturen (ausgefüllter Kreis 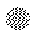 ) erfolgte mit der Software Aspen Properties V7.3 der Firma Aspen Technology. Die für die Berechnung benötigten thermodynamischen Eigenschaften von Ethen und Isobutan stammen aus der Stoffdatenbank APV73 mit PURE25, welches auf dem Release der DIPPR Datenbank (Januar 2010) basiert.

## Patentansprüche

1. Verfahren zur Oligomerisierung von Ethen, bei welchem ein Gemisch enthaltend Ethen sowie ein Inertmedium einen festen Katalysator kontaktiert,
**dadurch gekennzeichnet,**
**dass** der Gewichtsanteil des Inertmediums an dem Gemisch größer ist als der Gewichtsanteil des Ethens an dem Gemisch,
**dass** es sich bei dem Inertmedium um ein Alkan oder um ein Cycloalkan handelt, wobei das Alkan bzw. das Cycloalkan mindestens drei und maximal sieben Kohlenstoffatome aufweist,
und **dass** Druck und Temperatur des Gemisches in Hinblick auf den Anteil von Ethen in dem Gemisch so gewählt sind, dass Ethen und Inertmedium im überkritischen Zustand vorliegen.

2. Verfahren nach Anspruch 1, **dadurch gekennzeichnet, dass** das Gemisch zunächst im überkritischen Zustand bereit gestellt und sodann mit dem festen Katalysator kontaktiert wird, wobei die Bereitstellung des Gemisches im überkritischen Zustand entweder gemäß Alternative a) oder gemäß Alternative b) erfolgt:
a) Inertmedium wird durch Erhöhung von Druck und/oder Temperatur in den überkritischen Zustand überführt und Ethen wird in das überkritische Inertmedium unter Erhalt des überkritischen Gemisches eindosiert;
b) Inertmedium und Ethen werden zu dem Gemisch vermischt, und das Gemisch durch Erhöhung von Druck und/oder Temperatur in den überkritischen Zustand überführt.

3. Verfahren nach Anspruch 1 oder Anspruch 2, **dadurch gekennzeichnet, dass** es sich bei dem Inertmedium um eine der folgenden Substanzen handelt: Propan, Isobutan, n-Butan, Isopentan, n-Pentan, n-Hexan, n-Heptan, Cyclopentan, Cyclohexan, 2-Methylpentan, 3-Methylpentan, 2-Methylhexan, 3-Methylhexan, Methylcyclopentan, Methylcyclohexan.

4. Verfahren nach Anspruch 3, wobei es sich bei dem Inertmedium um Isobutan handelt,
**dadurch gekennzeichnet, dass** das der Anteil des Ethens im Gemisch zu Beginn des Kontakts mit dem Katalysator zwischen 4 Gew.-% und 30 Gew.-% beträgt, dass der Druck des Gemisches auf einen Wert zwischen 25*10⁵ Pa und 100*10⁵ Pa eingestellt ist, und dass die Temperatur des Gemisches auf einen Wert zwischen 90 °C und 200 °C eingestellt ist, unter der Maßgabe, dass sowohl Ethen als auch Isobutan im überkritischen Zustand vorliegen.

5. Verfahren nach Anspruch 4, **dadurch gekennzeichnet, dass** das Gemisch zusammen mit mindestens einem Begleitstoff als Eduktgemisch bereitgestellt wird, wobei das Eduktgemisch zum Beginn des Kontakts mit dem Katalysator die folgende, sich zu 100 Gew.-% ergänzende Zusammensetzung aufweist:
| | |
|---|---|
| Isobutan: | 70 Gew.-% bis 96 Gew.-%; |
| Ethen: | 4 Gew.-% bis 30 Gew.-%; |
| Summe aller Begleitstoffe: | mehr als 0 Gew.-% bis maximal 5 Gew.-% |

6. Verfahren nach Anspruch 4 oder 5, **dadurch gekennzeichnet, dass** von dem Katalysator ein Produktgemisch mit der folgenden, sich zu 100 Gew.-% ergänzenden Zusammensetzung abgezogen wird:
| | |
|---|---|
| Isobutan: | 70 Gew.-% bis 96 Gew.-%; |
| Ethen: | 0 Gew.-% bis 2 Gew.-%; |
| Olefine mit vier Kohlenstoffatomen: | 2.3 Gew.-% bis 21 Gew.-%; |
| Olefine mit sechs Kohlenstoffatomen: | 0.9 Gew.-% bis 7.2 Gew.-%; |
| Olefine mit acht Kohlenstoffatomen: | 0.1 Gew.-% bis 6.3 Gew.-%; |
| Olefine mit zehn Kohlenstoffatomen: | 0 Gew.-% bis 3 Gew.-%; |
| Olefine mit zwölf Kohlenstoffatomen: | 0 Gew.-% bis 2.7 Gew.-%; |
| Summe aller sonstigen Bestandteile: | 0 Gew.-% bis 5 Gew.-%. |

7. Verfahren nach Anspruch 3, wobei es sich bei dem Inertmedium um n-Hexan handelt, **dadurch gekennzeichnet, dass** der Anteil des Ethens im Gemisch zu Beginn des Kontakts mit dem Katalysator zwischen 5 Gew.-% und 30 Gew.-% beträgt, dass der Druck des Gemisches auf einen Wert zwischen 25*10⁵ Pa und 100*10⁵ Pa eingestellt ist, und dass die Temperatur des Gemisches auf einen Wert zwischen 90 °C und 250 °C eingestellt ist, unter der Maßgabe, dass sowohl Ethen als auch n-Hexan im überkritischen Zustand vorliegen.

8. Verfahren nach Anspruch 1 oder einem der Ansprüche 2 bis 7, **dadurch gekennzeichnet, dass** es sich bei dem Katalysator um einen Festkörper handelt, welcher mindestens zwei Komponenten enthält, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

9. Kombination eines Verfahrens nach Anspruch 1 oder einem der Ansprüche 2 bis 8 mit einem Verfahren zur Regeneration des Katalysators; mit den folgenden Merkmalen:
a) die Oligomerisierung erfolgt in einem Oligomerisierungs-Betrieb, in welchem Inertmedium und Ethen jeweils im überkritischen Zustand mit dem Katalysator kontaktiert werden;
b) die Regeneration erfolgt in einem Regenerations-Betrieb, in welchem der Katalysator in Abwesenheit von Ethen, Wasserstoff und Sauerstoff mit einem flüssigen Spülmedium gespült wird;
c) über die Zeit wird gewechselt zwischen dem Oligomerisierungs-Betrieb und dem Regenerations-Betrieb, sodass sich an einen zeitlich begrenzten Oligomerisierungs-Betrieb ein zeitlich begrenzter Regenerations-Betrieb anschließt, und an diesen wieder ein zeitlich begrenzter Oligomerisierungs-Betrieb;
d) der Katalysator befindet sich stets an demselben Ort, weswegen sowohl Oligomerisierungs-Betrieb als auch Regenerations-Betrieb an diesem Ort stattfinden;
e) der Ort, an dem sich der Katalysator befindet, wird mit positiver bzw. negativer Wärmenergie beaufschlagt, um ihm eine Vorgabe-Temperatur aufzuprägen, wobei die tatsächliche Temperatur am Katalysator zeitlich und örtlich begrenzt durchaus von der Vorgabe-Temperatur abweichen kann;
f) die Vorgabe-Temperatur im Regenerations-Betrieb ist niedriger als die Vorgabe-Temperatur im Oligomerisierungs-Betrieb;
g) der Druck an dem Ort, an dem sich der Katalysator befindet, ist im Regenerations-Betrieb niedriger als im Oligomerisierungs-Betrieb.

10. Kombination nach Anspruch 9, wobei als Spülmedium und Inertmedium dieselbe Substanz verwendet wird, unter der Maßgabe, dass sich diese Substanz im Oligomerisierungs-Betrieb im überkritischen Zustand befindet und im Regenerations-Betrieb im flüssigen Zustand.

11. Kombination nach Anspruch 10, wobei als Spülmedium und Inertmedium Isobutan verwendet wird, **dadurch gekennzeichnet, dass** der Druck im Oligomerisierungs-Betrieb auf einen Wert zwischen 45*10⁵ Pa bis 55*10⁵ Pa eingestellt ist und die Temperatur im Oligomerisierungs-Betrieb auf einen Wert zwischen 125 °C und 155 °C eingestellt ist, und dass im Regenerations-Betrieb Druck und Temperatur abgesenkt werden, nämlich auf einen Druck zwischen 25*10⁵ Pa und 35*10⁵ Pa bzw. auf eine Temperatur zwischen 105 °C und 125 °C.

## Claims

1. Process for oligomerizing ethene, in which a mixture comprising ethene and an inert medium contacts a solid catalyst,
**characterized in that**
the proportion by weight of the inert medium in the mixture is greater than the proportion by weight of ethene in the mixture,
the inert medium is an alkane or a cycloalkane, where the alkane or cycloalkane has not less than three and not more than seven carbon atoms,
and pressure and temperature of the mixture are chosen with respect to the proportion of ethene in the mixture such that ethene and inert medium are in the supercritical state.

2. Process according to Claim 1, **characterized in that** the mixture is at first provided in the supercritical state and then contacted with the solid catalyst, where the provision of the mixture in the supercritical state is affected either according to alternative a) or according to alternative b):
a) inert medium is converted to the supercritical state by increasing the pressure and/or temperature and ethene is metered into the supercritical inert medium to obtain the supercritical mixture;
b) inert medium and ethene are mixed to give the mixture, and the mixture is converted to the supercritical state by increasing the temperature and/or pressure.

3. Process according to Claim 1 or Claim 2, **characterized in that** the inert medium is one of the following substances: propane, isobutane, n-butane, isopentane, n-pentane, n-hexane, n-heptane, cyclopentane, cyclohexane, 2-methylpentane, 3-methylpentane, 2-methylhexane, 3-methylhexane, methylcyclopentane, methylcyclohexane.

4. Process according to Claim 3, where the inert medium is isobutane, **characterized in that** the proportion of ethene in the mixture on commencement of contact with the catalyst is between 4% by weight and 30% by weight, the pressure of the mixture is adjusted to a value between 25*10⁵ Pa and 100*10⁵ Pa, and the temperature of the mixture is adjusted to a value between 90°C and 200°C, with the proviso that both ethene and isobutane are in the supercritical state.

5. Process according to Claim 4, **characterized in that** the mixture is provided as reactant mixture together with at least one accompanying substance, where the reactant mixture on commencement of contact with the catalyst has the following composition that adds up to 100% by weight:
| | |
|---|---|
| Isobutane: | 70% by weight to 96% by weight; |
| Ethene: | 4% by weight to 30% by weight; |
| Sum of all accompanying substances: | more than 0% by weight to a maximum of 5% by weight. |

6. Process according to Claim 4 or 5, **characterized in that** a product mixture having the following composition that adds up to 100% by weight is drawn off from the catalyst:
| | |
|---|---|
| Isobutane: | 70% by weight to 96% by weight; |
| Ethene: | 0% by weight to 2% by weight; |
| Olefins having four carbon atoms: | 2.3% by weight to 21% by weight; |
| Olefins having six carbon atoms: | 0.9% by weight to 7.2% by weight; |
| Olefins having eight carbon atoms: | 0.1% by weight to 6.3% by weight; |
| Olefins having ten carbon atoms: | 0% by weight to 3% by weight; |
| Olefins having twelve carbon atoms: | 0% by weight to 2.7% by weight; |
| Sum of all other constituents: | 0% by weight to 5% by weight. |

7. Process according to Claim 3, where the inert medium is n-hexane, **characterized in that** the proportion of ethene in the mixture on commencement of contact with the catalyst is between 5% by weight and 30% by weight, the pressure of the mixture is adjusted to a value between 25*10⁵ Pa and 100*10⁵ Pa, and the temperature of the mixture is adjusted to a value between 90°C and 250°C, with the proviso that both ethene and n-hexane are in the supercritical state.

8. Process according to Claim 1 or any of Claims 2 to 7, **characterized in that** the catalyst is in the solid state and comprises at least two components, where the first component comprises at least one element selected from Ni, Cr, Fe, Ti which is in metallic and/or oxidic and/or hydridic form, and where the second component comprises at least one metal oxide selected from Al₂O₃, SiO₂, TiO₂, ZrO₂.

9. Combination of a process according to Claim 1 or any of Claims 2 to 8 with a process for regeneration of the catalyst, having the following features:
a) the oligomerization is effected in an oligomerization operation in which inert medium and ethene, each in the supercritical state, are contacted with the catalyst;
b) the regeneration is effected in a regeneration operation in which the catalyst in the absence of ethene, hydrogen and oxygen is purged with a liquid purge medium;
c) operation is interchanged over time between the oligomerization operation and the regeneration operation, in such a way that a time-limited oligomerization operation is followed by a time-limited regeneration operation, and the latter in turn is followed by a time-limited oligomerization operation;
d) the catalyst is always at the same location, which is the reason why both oligomerization operation and regeneration operation take place at this location;
e) the location of the catalyst is supplied with positive or negative thermal energy in order to impose a set temperature thereon, it being entirely possible for the actual temperature at the catalyst to deviate from the set temperature in a time- and space-limited manner;
f) the set temperature in regeneration operation is lower than the set temperature in oligomerization operation;
g) the pressure at the location of the catalyst is lower in regeneration operation than in oligomerization operation.

10. Combination according to Claim 9, where the purge medium and inert medium used is the same substance, with the proviso that this substance is in the supercritical state in oligomerization operation and in the liquid state in regeneration operation.

11. Combination according to Claim 10, where the purge medium and inert medium used is isobutane, **characterized in that** the pressure in oligomerization operation is adjusted to a value between 45*10⁵ Pa and 55*10⁵ Pa and the temperature in oligomerization operation is adjusted to a value between 125°C and 155°C, and pressure and temperature are lowered in regeneration operation, namely to a pressure between 25*10⁵ Pa and 35*10⁵ Pa and to a temperature between 105°C and 125°C.

## Revendications

1. Procédé pour l'oligomérisation d'éthylène, dans lequel un mélange contenant de l'éthylène ainsi qu'un milieu inerte est en contact avec un catalyseur solide, **caractérisé en ce que** la proportion pondérale du milieu inerte par rapport au mélange est supérieure à la proportion pondérale de l'éthylène par rapport au mélange,
**en ce qu'**il s'agit, pour le milieu inerte, d'un alcane ou d'un cycloalcane, l'alcane ou le cycloalcane présentant au minimum trois et au maximum sept atomes de carbone,
et **en ce que** la pression et la température du mélange, eu égard à la proportion d'éthylène dans le mélange, sont choisies de manière telle que l'éthylène et le milieu inerte se trouvent dans l'état surcritique.

2. Procédé selon la revendication 1, **caractérisé en ce que** le mélange est d'abord préparé dans l'état surcritique et ensuite mis en contact avec le catalyseur solide, la préparation du mélange dans l'état surcritique ayant lieu, soit selon l'alternative a), soit selon l'alternative b) :
a) le milieu inerte est amené dans l'état surcritique par augmentation de la pression et/ou de la température et l'éthylène est dosé dans le milieu inerte surcritique avec obtention du mélange surcritique ;
b) le milieu inerte et l'éthylène sont mélangés en mélange et le mélange est amené dans l'état surcritique par augmentation de la pression et/ou de la température.

3. Procédé selon la revendication 1 ou la revendication 2, **caractérisé en ce qu'**il s'agit, pour le milieu inerte, d'une des substances suivantes : propane, isobutane, n-butane, isopentane, n-pentane, n-hexane, n-heptane, cyclopentane, cyclohexane, 2-méthylpentane, 3-méthylpentane, 2-méthylhexane, 3-méthylhexane, méthylcyclopentane, méthylcyclohexane.

4. Procédé selon la revendication 3, où il s'agit, pour le milieu inerte, d'isobutane, **caractérisé en ce que** la proportion d'éthylène dans le mélange au début du contact avec le catalyseur est située entre 4% en poids et 30% en poids, **en ce que** la pression du mélange est réglée à une valeur entre 25*10⁵ Pa et 100*10⁵ Pa et **en ce que** la température du mélange est réglée à une valeur entre 90°C et 200°C à condition que tant l'éthylène que l'isobutane se trouvent dans l'état surcritique.

5. Procédé selon la revendication 4, **caractérisé en ce que** le mélange est préparé ensemble avec une substance accompagnante comme mélange de départ, le mélange de départ présentant, au début du contact avec le catalyseur, la composition suivante, se complétant à 100% en poids :
isobutane : 70% en poids à 96% en poids ;
éthylène : 4% en poids à 30% en poids ;
somme de toutes les substances accompagnantes : plus de 0% en poids à maximum 5% en poids.

6. Procédé selon la revendication 4 ou 5, **caractérisé en ce qu'**on soutire du catalyseur un mélange produit présentant la composition suivante, se complétant à 100% en poids :
isobutane : 70% en poids à 96% en poids ;
éthylène : 0% en poids à 2% en poids ;
oléfines comprenant quatre atomes de carbone : 2,3% en poids à 21% en poids ;
oléfines comprenant six atomes de carbone : 0,9% en poids à 7,2% en poids ;
oléfines comprenant huit atomes de carbone : 0,1% en poids à 6,3% en poids ;
oléfines comprenant dix atomes de carbone : 0% en poids à 3% en poids ;
oléfines comprenant douze atomes de carbone : 0% en poids à 2,7% en poids ;
somme de tous les autres constituants : 0% en poids à 5% en poids.

7. Procédé selon la revendication 3, où il s'agit, pour le milieu inerte, de n-hexane, **caractérisé en ce que** la proportion d'éthylène dans le mélange au début de la mise en contact avec le catalyseur est située entre 5% en poids et 30% en poids, **en ce que** la pression du mélange est réglée à une valeur entre 25*10⁵ Pa et 100*10⁵ Pa et **en ce que** la température du mélange est réglée à une valeur entre 90°C et 250°C à condition que tant l'éthylène que le n-hexane se trouvent dans l'état surcritique.

8. Procédé selon la revendication 1 ou selon l'une quelconque des revendications 2 à 7, **caractérisé en ce qu'**il s'agit, pour le catalyseur, d'un corps solide qui contient au moins deux composants, le premier composant comprenant au moins un élément, choisi parmi Ni, Cr, Fe, Ti, qui se trouve sous forme métallique et/ou oxyde et/ou hydrure, et le deuxième composant comprenant au moins un oxyde métallique choisi parmi Al₂O₃, SiO₂, TiO₂, ZrO₂.

9. Combinaison d'un procédé selon la revendication 1 ou selon l'une quelconque des revendications 2 à 8 avec un procédé pour la régénération du catalyseur ; présentant les caractéristiques suivantes :
a) l'oligomérisation a lieu dans un fonctionnement d'oligomérisation dans lequel le milieu inerte et l'éthylène, à chaque fois dans l'état surcritique, sont mis en contact avec le catalyseur ;
b) la régénération a lieu dans un fonctionnement de régénération, dans lequel le catalyseur, en l'absence d'éthylène, d'hydrogène et d'oxygène, est rincé avec un milieu de rinçage liquide ;
c) on alterne, dans le temps, entre le fonctionnement d'oligomérisation et le fonctionnement de régénération, de telle sorte qu'un fonctionnement d'oligomérisation limité dans le temps est suivi d'un fonctionnement de régénération limité dans le temps et celui-ci est de nouveau suivi d'un fonctionnement d'oligomérisation limité dans le temps ;
d) le catalyseur se trouve toujours au même endroit, suite à quoi tant le fonctionnement d'oligomérisation que le fonctionnement de régénération ont lieu en cet endroit ;
e) l'endroit où se trouve le catalyseur est soumis à une énergie thermique positive ou négative afin de lui conférer une température de consigne, la température effective au niveau du catalyseur pouvant absolument s'écarter de la température de consigne de manière limitée dans le temps et de manière localement limitée ;
f) la température de consigne dans le fonctionnement de régénération est inférieure à la température de consigne dans le fonctionnement d'oligomérisation ;
g) la pression à l'endroit où se trouve le catalyseur est inférieure, dans le fonctionnement de régénération, à celle dans le fonctionnement d'oligomérisation.

10. Combinaison selon la revendication 9, la même substance étant utilisée comme milieu de rinçage et comme milieu inerte, à condition que cette substance se trouve dans l'état surcritique dans le fonctionnement d'oligomérisation et dans l'état liquide dans le fonctionnement de régénération.

11. Combinaison selon la revendication 10, de l'isobutane étant utilisé comme milieu de rinçage et comme milieu inerte, **caractérisée en ce que** la pression dans le fonctionnement d'oligomérisation est réglée à une valeur entre 45*10⁵ Pa et 55*10⁵ Pa et la température dans le fonctionnement d'oligomérisation est réglée à une valeur entre 125°C et 155°C et **en ce que**, dans le fonctionnement de régénération, la pression et la température sont abaissées, à savoir respectivement à une pression entre 25*10⁵ Pa et 35*10⁵ Pa et à une température entre 105°C et 125°C.
